# EUROPÄISCHE PATENTSCHRIFT

(11) **EP 1 143 849 B1**
(45) Veröffentlichungstag und Bekanntmachung des Hinweises auf die Patenterteilung: **02.05.2003**
(21) Anmeldenummer: 00900482.1
(22) Anmeldetag: 24.01.2000
(51) Int. Cl.: A61B 3/135

(54) **OPHTHALMOLOGISCHES GERÄT MIT EINEM BELEUCHTUNGS- UND/ODER STRAHLENBEHANDLUNGSSTRAHL, DESSEN FLÄCHIGE INTENSITÄTSVERTEILUNG EINSTELLBAR IST, SOWIE ANORDNUNG MIT DIESEM GERÄT ZUR AUGENBEHANDLUNG**
OPHTHALMIC APPARATUS WITH A LIGHTING AND/OR BEAM THERAPY RAY WHOSE SURFACE INTENSITY DISTRIBUTION CAN BE REGULATED AND DEVICE WITH SUCH AN APPARATUS FOR EYE TREATMENT
APPAREIL OPHTALMOLOGIQUE A FAISCEAU D'ECLAIRAGE ET/OU DE TRAITEMENT PAR RAYONNEMENT, A REPARTITION REGLABLE D'INTENSITE EN SURFACE, ET DISPOSITIF ASSOCIE POUR LE TRAITEMENT OCULAIRE

(30) Priorität: 22.01.1999 CH 12699
(43) Veröffentlichungstag der Anmeldung: 17.10.2001
(73) Patentinhaber: HAAG-STREIT AG, CH-3098 Köniz (CH)
(72) Erfinder: ULBERS, Gerd, CH-3132 Riggisberg (CH)
(74) Vertreter: Roshardt, Werner Alfred, Dipl.-Phys.
(86) Internationale Anmeldenummer: CH0000032
(87) Internationale Veröffentlichungsnummer: WO00042901

(56) Entgegenhaltungen:
- EP-A- 0 482 340
- DE-A- 4 211 502
- US-A- 5 018 851
- US-A- 5 801 807

## Beschreibung

### Technisches Gebiet

Die Erfindung betrifft ein ophthalmologisches Gerät, insbesondere ein sog. Spaltlampengerät gemäss dem Oberbegriff des Patentanspruchs 1 und eine Anordnung gemäss Patentanspruch 10.

### Stand der Technik

Ein Spaltlampengerät als ophthalmologisches Gerät ist beispielsweise von der Firma Haag-Streit unter der Bezeichnung "Original-Spaltlampe 900 BM" bekannt. Ein weiteres Spaltlampengerät ist in der europäischen Patentanmeldung EP-A 0 916 306 (EP-98 810 895.7) beschrieben.

In der US-A 5 801 807 ist ein ophthalmologisches Gerät mit einem Flächenlichtmodulator beschrieben. Als Flächenlichlmodulator wurde ein Flüssigkristall oder auch andere optische Elemente wie elektrochrome Elemente verwendet, welche matrixartig zur örtlichen Änderung des Transmissionsverhaltens über eine elektronische Schaltung angesteuert wurden. Das mit dem Flächenlichtmodulator erzeugbare Bild wurde über einen Strahlteiler in den auf das menschliche Auge gerichteten Beobachtungsstrahlengang eingeblendet.

In der US-A 5 018 851 ist ein Spaltlampengerät mit einer optischen Einheit beschrieben, mit der ein auf dem Auge abzubildender Schlitz über das Auge bewegbar war bzw. ein variabler Einstrahlwinkel erzeugt wurde. Die optische Einheit bestand aus einem elektrisch in seiner Transmission ein- und ausschaltbaren Flüssigkristall bestehen, dem ein bewegbarer Schlitz bzw. eine Schlitzanordnung vorgeschaltet war. Auch konnten jedoch einzelne Spalten auf einem Flüssigkristall transparenzmäßig ein- und ausgeschaltet werden.

In der EP-A 0 482 340 ist ein ophthalmologisches Mikroskop mit einem veränderbaren Verschluss im Beleuchtungssystem beschrieben. Dieser veränderbare Verschluss war eine rotierende Scheibe mit einer Öffnung als Kreissegment. Die Scheibe war auch durch einen elektronisch ansteuerbarer Flüssigkristall ersetzbar. Der veränderbare Verschluss diente hier nicht zur Erzeugung einer Bildinformation.

### Darstellung der Erfindung

### Aufgabe der Erfindung

Aufgabe der Erfindung ist es, ein ophthalmologisches Gerät, insbesondere ein sog. Spaltlampengerät zu schaffen, welches gegenüber den bekannten Geräten dem behandelnden bzw. untersuchenden Augenarzt einen bedeutend grösseren Anwendungsbereich in ein- und demselben Gerät mit äusserst einfacher Bedienung zur Verfügung stellt. Ferner soll dieses Gerät in eine Anordnung zur Augenbehandlung integrierbar sein.

### Lösung der Aufgabe

Die Lösung der Aufgabe ist Gegenstand des Patentanspruchs 1:

Die Erfindung weist nämlich gegenüber den bekannten Geräten nicht mehr eine mechanische Blende auf, mit der die geometrische Querschnittsform eines untersuchenden Lichtstrahls einstellbar ist. Die querschnittsmässige Intensitätsverteilung eines für die Augenuntersuchung benötigten Lichtstreifens (Spalt) wird nun erfindungsgemäss mit einem sog. Flächenlichtmodulator vorgenommen. Die einstellbaren optischen Eigenschaften dieses Flächenlichtmodulators werden an vorgebbaren und frei wählbaren Orten mit den unten angeführten Vorgehensweisen auf eine einfache Art und Weise eingestellt bzw. verändert. Die optischen Eigenschaften über den durchstrahlbaren Bereich des Flächenlichtmodulators sind nun - ferngesteuert - derart veränderbar, dass eine gewünschte optische Struktur mit vorgebbaren optischen Eigenschaften erreicht wird. Diese optische Struktur verändert nun einen auf sie fallenden Lichtstrahl entweder in Transmission oder Reflexion entsprechend der geforderten Intensitätsverteilung. Diese unterschiedliche Intensitätsverteilung hat auf bzw. im Auge im allgemeinen bis auf unten angeführte Unterschiede die gleiche Wirkung wie die bisher verwendeten mechanischen Blenden. Es kann jetzt der geometrische Querschnitt eines Beleuchtungsstrahls auf einfache Art und Weise schnell verändert werden. Es kann aber ebenso einfach aus diesen ein Lichtband, eine Linienanordnung, eine Punkteanordnung oder eine sonstige optische Figuren für die Augenbeleuchtung bzw. - untersuchung erzeugt, d.h. seine Intensitätsverteilung geändert werden,

Je nach verwendetem, unten beschriebenen Flächenlichtmodulator können Intensitätsverteilungen d.h. Bilder erzeugt werden, deren einzelne Bildelemente eine Rasterung aufweisen oder rasterfrei sind, wobei unter einer Rasterung, analog zur allgemeinen Drucktechnik eine gleich distanzierte Punkteanordnung verstanden wird. Die distanzierte Punkteanordnung kann selbstverständlich unterschiedlich sein; ist aber in ein- und demselben Bild immer gleich. Eine Rasterung wird immer dann erhalten, wenn Bildelemente in einem Flächenlichtmodulator durch eine matrixartige, in der Regel elektrische Ansteuerung aktiviert werden. Unter einer Rasterung wird hier jedoch nicht eine Punkteanordnung verstanden wie sie untenstehend zur Bestimmung von Abbildungsfehlern des Auges verwendet wird.

Mit einem rasterfrei arbeitenden Flächenlichtmodulator lassen sich im Gegensatz zu einem mit einer Rasterung arbeitenden Flächenlichtmodulator z.B. flächige Bildelemente mit einer konstanten optischen Eigenschaft ohne innere Struktur erzeugen.

Eine rasterfreie Erzeugung von Bildelementen wird z.B. dann erreicht, wenn ein entsprechendes Bild optisch mit einem Licht- bzw. Laserstrahl oder einer projizierten Abbildung in den Flächenlichtmodulator "eingeschrieben" bzw. "eingeprägt" wird. Die "Schreibstrahlen" können hier in Abhängigkeit ihrer Intensitätsverteilung über dem Strahlquerschnitt, derart aneinander im Strahlrandbereich überlappend bewegt werden, dass mit dem jeweils beschriebenen Flächenbereich ein Bildelement mit konstanter Leuchtdichte erzeugbar ist. Selbstverständlich können auch variable Leuchtdichten erzeugt werden. Es können neben den unten erwähnten optischen Figuren in einer Helldunkeldarstellung auch Figuren mit unterschiedlichen Grautönen erzeugt werden.

Eine Unterscheidung in der Verwendung zwischen diesen beiden Typen von Flächenlichtmodulatoren ist wichtig, da hierdurch unterschiedliche Empfindungen im Auge erzeugt und damit auch unterschiedliche Testverfahren am Auge vorgenommen werden können. Für einen zu untersuchenden Betrachter ergibt sich nämlich ein vollkommen unterschiedlicher Eindruck bei in sich gerasterten optischen Figuren und Figuren mit einem rasterfreien Intensitätsverlauf.

Der ophthalmologische Fachmann war bisher bei den zu Untersuchungen verwendeten bekannten Spaltlampengeräten immer von einer einstellbaren mechanischen Blende ausgegangen. Eine Verwendung einer derartigen Blende war naheliegend, da auf dem Auge bzw. innerhalb jeweils ein Lichtband mit einem umliegenden dunklen Bereich abgebildet werden sollte, was eben mit einer mechanischen Blende, wie z. B. auch bei Fotoapparaten, erzeugt wurde. Sollten abweichend von einem einfachen Lichtband für keratometrische Messungen geometrische Strukturen auf bzw. im Auge abgebildet werden, so wurden auch hier, wie in der US-A 5 418 582 beschrieben, mechanische Blenden mit vollständig durchscheinenden und vollständig abblockenden Blendenbereichen verwendet. Mit der unten beschriebenen Erfindung kann auf einfache Art und Weise ohne jegliche Hardwareänderung beispielsweise auch die mit dem Ringkonus der US-A 5 418 582 erzeugte geometrische Struktur neben einer üblichen Lichtbandabbildung abgebildet werden.

Mit einem derartigen Flächenlichtmodulator kann nun ein "Lichtband" mit Abmessungen und einer Intensitätsverteilung erzeugt werden, wie sie dem herkömmlichen entspricht, welches mit einer mechanisch einstellbaren Blende vorgenommen worden ist. Über ein derartiges "Lichtband" hinaus können jetzt jedoch beliebige Intensitätsverteilungen und -figuren erzeugt werden. Man kann nun z. B. eine Vielzahl parallel zueinander liegende oder unter beliebigen Winkeln zueinander liegende Lichtbänder erzeugen. Auch können Kreisringe, Vollkreise, Placido-Ringe, Kreuzgitter, ... erzeugt werden.

Da aus dem Beobachtungsstrahlengang Bildinformationen für eine Videoaufnahmeeinheit ausblendbar sind, wie insbesondere in der bereits erwähnten EP-A 0 916 306 (EP-98 810 895.7) beschrieben, kann zudem eine automatische Auswertung vorgenommen werden. Das ursprüngliche Bild kann mit seiner Projektion auf gekrümmten Flächen wie der Hornhaut und der Retina verglichen werden. Aus diesem Vergleich können dann die entsprechenden Krümmungen automatisch von einer Auswerteeinheit bestimmt werden.

Das erfindungsgemässe Gerät eignet sich somit ausgezeichnet neben den bereits bekannten Untersuchungen und Behandlungsmethoden eines Spaltlampengeräts zur Comea-Dickenmessung, zur Erstellung eines 2D- oder 3D-Fundusprofils, zur Corneatopographie, zur Fundustopographie, ....

### Kurze Beschreibung der Zeichnungen

In den folgenden Figuren werden Geräte gezeigt, welche das Verständnis der Erfindung erleichtern sollen. Es zeigen:
- Fig. 1: eine Seitenansicht eines ophthalmologischen Geräts als Spaltlampengerät, welches sich in seinem Äusseren nur geringfügig von einem bereits bekannten Spaltlampengerät unterscheidet, jedoch einen Flächenlichtmodulator für den Augenbeleuchtungsstrahl aufweist; eine an die Betrachtungseinheit anflanschbare Videoaufnahmeeinheit ist hier nicht eingesetzt; der Anflanschort ist mit einem Stopfen **2** verschlossen,
- Fig. 2: ein Blockschema eines Ausführungsbeispiels für eine Beleuchtungseinheit des in **Figur 1** dargestellten Geräts, wobei ein rasterbehafteter Flächenlichtmodulator in Transmission betrieben wird und
- Fig. 3: ein Blockschema mit einer Variante zur Darstellung in **Figur 2,** wobei hier der Flächenlichtmodulator in Reflexion betrieben wird; die mit einem verschwenkbaren Linsenhalter wie in **Fig. 2** ein- und ausschwenkbare Zusatzlinse ist hier nicht mehr dargestellt.
Im folgenden werden Beispiele des erfindungsgemässen opthalmologischen Geräts anhand der nachfolgenden Zeichnungen näher erläutert. Es zeigen:
- Fig. 4: einen zur Herstellung des erfindungsgemässen Geräts integrierbaren Flächenlichtmodulator, in den eine rasterfreie "Blendeninformation" mit einem Licht- bzw. Laserstahl einschreibbar ist, und
- Fig. 5: eine Variante zur Erzeugung einer rasterfreien "Blendeninformation", bei der mit dem Flächenlichtmodulator örtlich die Polarisationsrichtung transmittierter Strahlung drehbar ist.

### Wege zur Ausführung der Erfindung

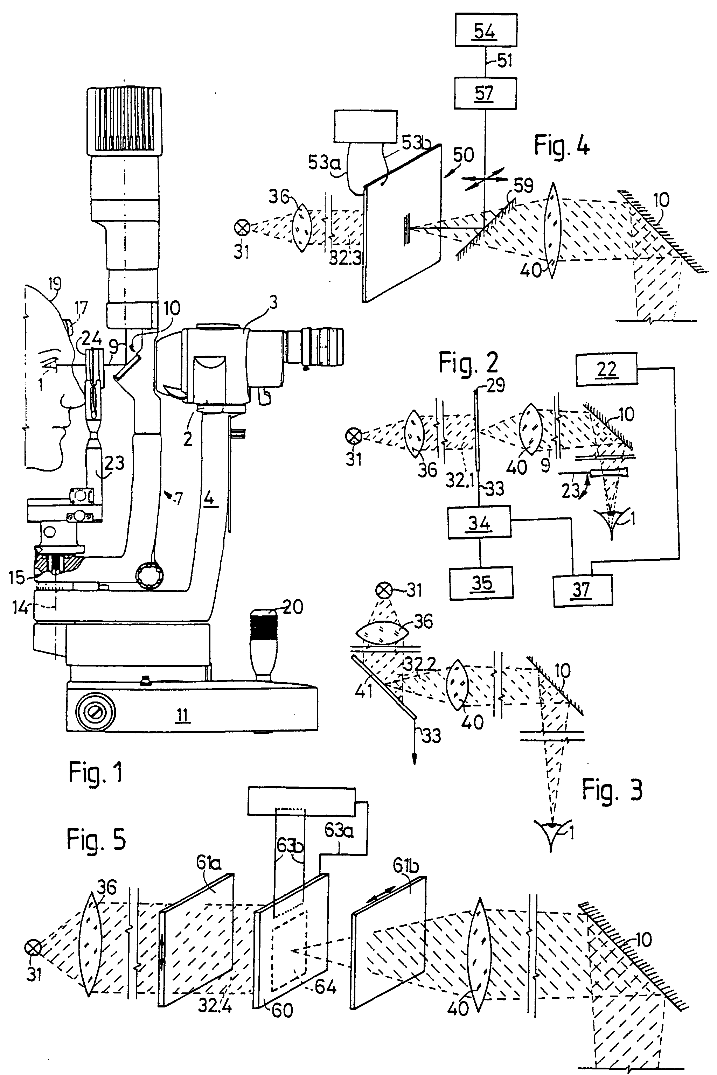

Das in der **Figur 1** dargestellte ophthalmologische Gerät ist als sog. Spaltlampengerät ausgebildet und dient zur stereoskopischen Betrachtung eines menschlichen Auges **1.** Das Spaltlampengerät hat eine Betrachtungseinheit**3** und eine Beleuchtungseinheit **5.** Die Betrachtungseinheit **3** ist mit einer L-förmigen Halteeinheit **4** und die Beleuchtungseinheit **5** mit einer weiteren L-förmigen Halteeinheit **7** gehalten. Von der Beleuchtungseinheit **5** wird ein Lichtstrahl **9** ausgesandt, der über einen an der Halteeinheit **7** angeordneten Umlenkspiegel **10** in bzw. auf das Auge **1** führbar ist. Die Intensitätsverteilung des Lichtstrahls **9** über seinem Querschnitt wird mit einem Flächenlichtmodulator, dessen Wirkungsweise unten beschrieben ist, eingestellt. Die Halteeinheit **7** ist über ein Schwenkgelenk **15** mit einer vertikalen Schwenkachse **14** verschwenkbar an einem Vorrichtungsfuss **11** angeordnet. Die Lage der Achse **14** ist derart gewählt, dass sie bei einer an ein (zeichnerisch nur angedeutetes) Stirnband **17** eines (nicht dargestellten) Kopfhalters angelegten menschlichen Stim **19** an der Augenvorderseite vorbei läuft.

Die Betrachtungseinheit **3** kann als Greenough-Mikroskop ausgebildet sein, aus dessen Strahlengang Bildinformationen in eine (nicht dargestellte) Videoaufnahmeeinheit lenkbar wären, wie insbesondere in der oben zitierten EP-A 0 916 306 (EP-98 810 895.7) ausführlich beschrieben ist. Es können somit neben einer visuellen Betrachtung zusätzlich Videoaufnahmen mit einer Videoeinheit **22** zur direkten Betrachtung, zur Aufzeichnung (Dokumentation) bzw. automatischen Auswertung vorgenommen werden.

Zwischen Umlenkspiegel **10** und dem zu untersuchenden bzw. zu behandelnden menschlichen Auge ist hier beispielsweise für die Betrachtung des Augenhintergrundes eine Abbildungslinse **21** auf einem in das Schwenkgelenk **15** einsteckbaren Linsenhalter **23** mit einer Zusatzlinse **24** angeordnet. Mit dieser Anordnung kann der Augenhintergrund betrachtet werden. Die Ausgestaltung dieses Linsenhalters **23** ist ebenfalls in der EP-A 0 916 306 (EP-98 810 895.7) ausführlich beschrieben. Erfolgt eine Betrachtung der Augenhornhaut, wird die Zusatzlinse **24** mit ihrem Linsenhalter **23** weggeschwenkt. Die Positionierung der Vorrichtung horizontal in X- und Y-Richtung wird mit Hilfe eines am Vorrichtungsfuss **11** angeordneten Lenkhebels **20,** oftmals auch als "Joy-Stick" bezeichnet, vorgenommen.

### Flächenlichtmodulatoren zur Erleichterung des Verständnisses der Erfindung

Ein Blockschema eines Ausführungsbeispiels für eine Beleuchtungseinheit **5** zur Betrachtung des Augenhintergrunds zeigt **Figur 2.** In diesem Ausführungsbeispiel wird ein Flächenlichtmodulator **29** als Einstellmittel für die Intensitätsverteilung eines von einer Lichtquelle **31** ausgesandten Lichtstrahls **9** über dessen Querschnitt verwendet. Die Begriffe Lichtstrahl und Lichtquelle sind hier etwas weiter gefasst, indem nicht unbedingt auf sichtbares Licht abgestellt wird. Unter Lichtquelle **31** werden hier auch Laserlichtquellen verstanden. Der Flächenlichtmodulator **29,** dessen Ausgestaltung unten ausgeführt wird, wird hier in Transmission verwendet. Der Flächenlichtmodulator **29** ist über elektrische Signalleitungen **33** mit einer Ansteuerelektronik **34** verbunden. Je nach Signalen auf den Ansteuerleitungen **33** werden hier auf dem Flächenlichtmodulator unterschiedliche Mikrostrukturbereiche derart angesteuert, dass sich ihre örtliche Transmissionsverteilung für die Strahlung der Lichtquelle **31** ändert. Die Ansteuerelektronik **34** ist signalmässig mit einer Eingabeeinheit **35** und mit einer Auswerteelektronik **37** verbunden, welche ihrerseits mit der Videoeinheit **22** signalmässig verbunden ist. Die angesteuerten Mikrostrukturbereiche des Flächenlichtmodulators **29** werden mit einem von der Lichtquelle **31** ausgesandten Lichtstrahl **32.1,** der beispielsweise mit einer Kollimatorlinse **36** zu einem kollimierten Strahl geformt ist, gleichmässig beleuchtet. Je nach ausgewählter Ansteuerung der betreffenden Mikrostrukturbereiche durch die Ansteuerelektronik **34** wird dann die gewünschte Transmissionsverteilung im flächigen Modulator **29** erreicht. Die aus dem Flächenlichtmodulator **29** austretende Strahlung, welche die Bildinformation des angesteuerten Modulators **29** enthält, wird mit einem Abbildungssystem **40** auf die Augenhomhaut fokussiert, oder unter Zwischenschaltung, wie in **Figur 2** dargestellt, auf den Augenhintergrund unter Verwendung der Zusatzlinse **24** fokussiert. Die Abbildung wird dann mit der Betrachtungseinheit **3** betrachtet bzw. über die Signale der Videoeinheit mit der Auswerteelektronik **37** ausgewertet.

Anstelle, wie in **Figur 2** gezeigt, den Flächenlichtmodulator **29** in Transmission zu betreiben, kann auch ein anders aufgebauter Flächenlichtmodulator **41**, wie in **Figur 3** angedeutet, in Reflexion betrieben werden.

### Flächenlichtmodulatoren zur Integration, um das erfindungsgemässe opthalmologische Gerät zu bilden

Neben einer elektrischen flächenmässigen Ansteuerung wird nun erfindungsgemäss mit einer Hilfsstrahlung eine Bildinformation auf einem Flächenlichtmodulator eingeprägt (eingeschrieben). Die Wellenlänge der einschreibenden Strahlung unterscheidet sich dann von der Strahlung, mit der die eingeschriebene Bildinformation ausgelesen, d.h. auf bzw. in das Auge abgebildet wird. Bei der Verwendung dieser Hilfsstrahlung kann je nach Aufbau des Flächenlichtmodulators in Transmission oder in Reflexion gearbeitet werden.

Einen Flächenlichtmodulator **50,** der in Transmission betrieben wird und in dem eine Bildinformation mit einem Laserstrahl **51** einschreibbar ist, zeigt **Figur 4.** Als Flächenlichtmodulator **50** kann ein in der DE-A 42 11 502 beschriebener Flüssigkeitskristall (nematische Phase) mit in ihm verteilten Festkörpern verwendet werden. Der Flüssigkristall ist hier, da in Transmission gearbeitet wird, zwischen zwei transparenten Platten, bevorzugt aus Glas, angeordnet, auf denen ebenfalls transparente Elektroden **53a** und **53b** aufgedampft sind. Verwendbare Flüssigkeitskristalle und die in sie einzulagernden Festkörper sowie Herstellungsverfahren hierzu sind in der DE-A 42 11 502 beschrieben. Zur lokalen Änderung optischer Eigenschaften im Flächenlichtmodulator **50** ist dieser mit einem von einer Laserquelle **54** aussendbaren Laserstrahl **51** überstreichbar. Der Laserstrahl **51** ist flächig mit einer Strahlablenkeinheit **57** ablenkbar. Die Intensität des Laserstrahls **51** kann über die Anregung der Laserquelle, über einen (nicht dargestellten) resonator-internen oder -externen Schalter ein- und ausgeschaltet. bzw. dessen Intensität eingestellt werden. Der Flächenlichtmodulator **50** arbeitet rasterfrei.

Vor dem Einschreiben einer Bildinformation wird man vorzugsweise zuerst eine homöotrope molekulare Ausrichtung der Moleküle des Flüssigkeitskristalls vornehmen. Diese Ausrichtung kann z.B. dadurch erfolgen, dass gemäss DE-A 42 11 502 an die Elektroden **53a** und **53b** eine Wechselspannung mit beispielsweise 500 Hz mit 100 V_{eff} angelegt wird. Dieselbe Ausrichtung kann jedoch bei einer niedrigeren Spannung bei gleichzeitigem Bestrahlen mit der Strahlung eines Halbleiterlasers erreicht werden. Eine Bildinformation wird dann bei spannungslosen Elektroden **53a** und **53b** mit einer höheren Laserleistung eingeschrieben. Die eingeschriebenen Bildelemente zeichnen sich dann gegenüber den nicht eingeschriebenen durch eine stark Iichtstreuende Wirkung aus.
Wird nun der Laserstrahl **51** mit der Strahlablenkeinheit **57** derart abgelenkt, dass eine Reihe von Linien sich an den Linienrändern überdeckend "geschrieben" werden, so kann entsprechend der Anzahl Linien und ihrer Länge ein Band mit vorgegebener Breite und Länge (Höhe) [entsprechend der Wirkung einer mechanisch verstellbaren Blende] mit zu erzeugender, vorgegebener bzw. mit einer einen vorgegebenen Verlauf aufweisenden, jedoch rasterfreien Leuchtdichte eingeschrieben werden. Nach dem "Einschreiben" wird der Flächenlichtmodulator **50** analog zum optischen Aufbau der **Figur 2** mit einer Strahlung **32.3** beleuchtet. Eine Abbildung des Flächenlichtmodulators **50** mit dem Abbildungssystem **40** ergibt dann je nachdem ob die Linse **24** verwendet wird, eine Abbildung eines Lichtbandes auf der Hornhaut oder der Netzhaut. Die Breite und Länge des Lichtbandes kann dann durch Löschen der eingeschriebenen Bildinformation im Flüssigkeitskristall, wie oben beschrieben, vollständig gelöscht und wieder neu mit anderen geometrischen Abmessungen eingeschrieben werden.

Es kann jedoch auch nur ein teilweises Löschen einer bereits eingeschriebenen Bildinformation vorgenommen werden, wenn an die Elektroden eine niedrigere Wechselspannung angelegt wird und der zu löschende Bereich mit dem Laserstrahl überstrichen wird. Spannungswerte und Intensitätswerte für den Laserstrahl bzw. Belichtungszeiten hängen von der Wellenlänge des Lasers und dem verwendeten Flüssigkeitskristallmaterial ab. Eine Auswahl von Daten kann der DE-A 42 11 502 entnommen werden.

Als Strahlablenkeinheit **57** können Spiegelchen oder ein bzw. zwei akustooptische Ablenker verwendet werden. Der Laserstrahl kann zum Einschreiben einer Bildinformation bzw. zu deren teilweisen Löschen auf die Oberfläche des Flächenlichtmodulators, welche der Lichtquelle zugewandt ist, gerichtet werden, oder, wie in **Figur 4** dargestellt, von der anderen, gegenüberliegenden Seite aus. Wird auf die gegenüberliegende Seite eingestrahlt, so muss der Laserstrahl entweder schräg auf diese Oberfläche gerichtet werden, damit er dann später nicht die Abbildung stört, oder über einen vergüteten Spiegel **59,** der die Strahlung des Lasers reflektiert, aber diejenige der Lichtquelle **31** transmittiert, auf die gegenüberliegende Seite des Flächenlichmodulators eingestrahlt werden (siehe **Fig. 4**). Die zuletzt genannte Anordnung hat den Vorteil, dass die einschreibende Laserstrahlung niemals ins zu untersuchende Auge gelangen kann.

In der DE-A 20 32 212 ist ein weiterer Flächenlichtmodulator beschrieben, der analog zu demjenigen der DE 42 11 502 einsetzbar ist. Auch hier wird ein Flüssigkeitskristall verwendet, dessen Material eine dichroitische Substanz beigemischt ist (z.B.p-n Butoxybenzoesäure als nematischer Flüssigkristall und Methylrot als dichroitischer Farbstoff). Dieser Flächenlichtmodulator ist eine Platte mit sandwichartigem Aufbau. An den Aussenseiten ist je eine transparente flächige Elektrode angeordnet. Wird nun im Gegensatz zu der Ausführung in der DE-A 20 32 212 eine der flächigen Elektroden derart ausgebildet, dass eine elektrische Ansteuerung von an vorgegebenen Orten angeordneten Teilelektroden möglich ist, so kann sich dann lediglich zwischen diesen jeweils angesteuerten Teilelektroden und der gegenüberliegenden ausgedehnten Elektrode eine Ausrichtung der dazwischen liegenden Moleküle erfolgen, wodurch die Transmission dieses Mikrostrukturbereichs gegenüber den "nicht angesteuerten" erhöht wird.

Anstelle optische Informationen in einen Flüssigkeitskristall insbesondere optisch und damit rasterfrei einzuschreiben, kann ein Einschreiben auch in eine elektro-optische Keramik eines Flächenlichtmodulators erfolgen. Hierzu verwendbare Materialien sind in der DE-A 24 16 684 beschrieben. Ein schnelles Einschreiben sowie auch Löschen einer Bildinformation ist möglich. Das elektro-optische keramische Material des Flächenlichtmodulators, in dem eine ferroelektrische Phase und eine antiferroelektrische Phase oder paraelektrische Phase nebeneinander mit einer morphotropen Phase vorliegen können, können feste Lösungen der allgemeinen Formel

Pb_{(1-x-α})LaₓA_{α}(Zr_{(1-y-β)}Ti_{y}M_{β})_{[1-((x+α)/4)]}O₃

sein. Es sollten die in der Formel aufgeführten Parameter x, α, y,β, A und M gemäss nachfolgenden Angaben ausgewählt werden:
0 ≤ x ≤ 0,2;
0 ≤ α < 1;
0 ≤ y ≤ 0,6;
0<β<1,
0<(y+β)≤1,
A Steht für mindestens ein Element aus der Gruppe der zweiwertigen und dreiwertigen Erdalkali- und Seltenenerdelemente;
M bedeutet mindestens ein Element aus der Gruppe der vierwertigen und fünfwertigen Metalle.

Der elektro-optische Kristall hat eine Dicke von etwa 200 µm. Auf seiner Vorder- und Hinterseite sind elektrisch leitende, optisch transparente Elektroden (z.B. aus SnCl₄)**63a** und **63b** aufgebracht. Sollen nur bereits vorbekannte Bilder erzeugt werden, so können die Elektroden mit den entsprechenden geometrischen Abmessungen der zu erzeugenden Bilder aufgebracht werden. Soll eine grosse Variationsmöglichkeit von Bildern erreicht werden, so wird eine Vielzahl einzeln ansteuerbarer Teilelektroden aufgebracht. Eine Elektrode **63a** der beiden Elektroden **63a** und **63b** erstreckt sich über die gesamte Oberfläche des zum Einschreiben von Informationen vorgesehenen Feldes **64.** Die auf der anderen Seite liegende Elektrodenanordnung z.B. **63b** weist dann die gewünschte Strukturierung auf. Zum Einschreiben eines Bildes wird beispielsweise bei Pb_{0,914}La_{0,084}A_{α}(Zr_{0,65}Ti_{0,35})_{0,979}O₃ an ausgewählte Teilelektroden der Elektrodenanordnung **63b** eine Spannung von 80V zwischen ihr und der flächigen Elektrode **63a** angelegt. Unter diesen angesteuerten Teilelektroden entsteht dann eine ferroelektrische Phase im elektro-optischen Kristall; es entsteht ein Bereich, der eine Drehung der Polarisationsebene (Doppelbrechung) von auf diesen Bereich fallendem Licht bzw. dessen Streuung hervorruft. In Bereichen, welche in der antiferroelektrischen Phase bzw. der paraelektrischen Phase verbleiben, erfolgt keine Streuung bzw. keine Doppelbrechung von eingestrahltem Licht. Diese Eigenschaft der angesteuerten Bereiche bleibt auch nach Abschalten der Spannung erhalten, sofern für das betreffende Material des Flüssigkeitskristalls eine typische Stabilitätstemperatur **Th** nicht überschritten wird. Je nach verwendetem Material liegt diese Temperatur **Th** zwischen 25°C und 70°C. Eine Materialauswahl hierzu gibt die DE-A 24 16 684 an.

Befindet sich nun ein derart ausgebildeter Flächenlichtmodulator **60** zwischen zwei Polarisatoren **61a** und **61b** (Polarisator **61a,** Analysator **61b**), deren Polarisationsrichtungen gekreuzt sind, wie in **Figur 5** angedeutet ist, so werden nur die angesteuerten Mikrostrukturbereiche durch das optische Abbildungssystem **62,** je nachdem, ob die Zusatzlinse **24** verwendet wird, auf der Augenhornhaut oder der Retina abgebildet.

Neben den für das Einschreiben bzw. Einprägen einer Bildinformation notwendigen Elektrodenanordnungen **63a** und **63b** sind ausserhalb des Feldes **64** Elektroden angeordnet, mit denen ein Stromfluss, bevorzugt ein pulsierender Stromfluss, einprägbar ist und der zu einer Erwärmung des Kristalls führt, welche deutlich über der obengenannten Stabilitätstemperatur **Th** liegt. Die "Löschelektrodenanordnung" ist derart zu wählen, dass im Informationsfeld **64** ein äusseres elektrisches Feld mit einer Polarität herrscht, welche der "eingeschriebenen Polarisation" entgegengesetzt ist, während das Feld **64** gleichzeitig erwärmt wird.

Die Elektrodenanordnung **63a** kann nun durch eine Photoleiterschicht ersetzt werden und gemäss obiger Ausführung zu **Figur 4** mit einem Lichtstrahl bzw. einem Laserstrahl "beschrieben" werden. Eine derart eingeprägte Information kann dann ebenfalls auf die Augenhornhaut oder den Augenhintergrund abgebildet werden.

Es kann jedoch auch ein Flächenlichtmodulator **41** unter Verwendung einer in der DE-C 20 11 575 beschriebenen Bildwiedergabevorrichtung ausgeführt werden. Neben der bereits in der DE-A 24 16 684 beschriebenen Anordnung mit einem nematischen Flüssigkeitskristall ist hier eine schlierenoptische Abbildung aufgezeigt. Mit einer kammartigen Elektrodenanordnung wird auf einem in der Dicke verformbaren Medium (z.B. eine auf einer Oberfläche haftende polymerisierte Siliconölschicht) wellenartig verformt. Diese wellenartig verformte Schicht wird von einem eine streifenartige Struktur aufweisenden Projektionslichtbündel unter einem derartigen Winkel von "hinten" bestrahlt, dass Totalreflexion eintritt. Das totalreflektierte Projektionslichtbündel mit seiner Streifenstruktur, der eine Wellstruktur überlagert ist, trifft auf eine streifenartige Blende, deren Streifen derart angeordnet sind, dass kein Licht des Projektionsbündels hindurch treten kann. In einem geringen Abstand von dieser verformbaren Schicht ist ein Photoleiter angeordnet. Wird dieser Photoleiter belichtet, so entsteht auf ihm eine der Belichtung entsprechende Ladungsverteilung, welche dann zusätzlich die verformbare Schicht verformt, wodurch der Verformung entsprechend Projektionslicht durch die streifenartige Blende entsprechend der Bildinformation tritt (=schlierenoptische Abbildung). In Weiterentwicklung der DE-C 20 11 575 kann jedoch der Photoleiter durch ein aufgedampftes, elektrisch leitendes Gitter ersetzt werden, dessen Gitterpunkte dann entsprechend von der Ansteuerelektronik zur Erzeugung des notwendigen Ladungsmusters mit der gewünschten Bildinformation angesteuert wird. Die zu verwendende optische Anordnung entspricht dann der in **Figur 3** gezeigten. Auch mit diesem Flächenlichtmodulator lassen sich rasterfreie Bilder erzeugen.

In Reflexion kann der Flächenlichtmodulator der DE-A 196 24 276 verwendet werden. Der hier beschriebene Flächenlichtmodulator arbeitet mit einer Phasenmodulation von an einem Spiegel reflektiertem Licht. Durch diese Phasenmodulation wird dem Licht die gewünschte Bildinformation, wie sie in dem oben beschriebenen ophthalmologischen Gerät für die Augenuntersuchung benötigt wird, aufgeprägt. Als Spiegel wirken mehrere dielektrische Festkörperschichten zusammen mit einer verformbaren plastischen dielektrischen Oberschicht. Diese Oberschicht kann eine dielektrische Flüssigkeit, beispielsweise ein Siliconöl, sein. Die dielektrische Flüssigkeit befindet sich nun innerhalb einer Elektrodenrasteranordnung, deren Rasterung den gewählten Mikrostrukturbereichen entspricht. Erfolgt nun eine elektrische Ansteuerung eines der Rasterpunkte, so ergibt dies eine Dickenveränderung der plastischen dielektrischen Schicht, wodurch dann nicht mehr die Phasenbeziehung für eine totale Lichtspiegelung gegeben ist. Dieser Bildpunkt ist somit abbildbar.

Die Grösse der zu erzeugenden Bereiche mit vorgegebenen optischen Eigenschaften des Flächenlichtmodulators und deren Dichte richtet sich nach den Abbildungsverhältnissen und der gewünschten geometrischen Auflösung. Für eine Funktion einer oben angeführten "Blendensimulation" bzw. Erzeugung eines vorgegebenen geometrischen Musters auf dem Auge, der Retina oder anderen gekrümmten Flächen, kann gemäss obigen Ausführungen ein Flächenlichtmodulator verwendet werden, dessen Bildbereiche entweder in Reflexion oder in Transmission für die abbildende Strahlung (Licht) arbeiten.

Wird mit Reflexion gearbeitet, so kann der Reflexionsfaktor, der Transmissionsfaktor sowie die Phasenlaufzeit vor einem rückwärtigen Spiegel bzw. die Reflexionsrichtung (Schlierenoptik) geändert werden. Erfolgt eine Auslegung auf Transmission, so kann ebenfalls schlierenoptisch gearbeitet werden sowie eine unterschiedliche Absorption, eine unterschiedliche Phasenlaufzeit, sowie ein unterschiedlicher Drehwinkel der Polarisation des transmittierenden Lichtes vorgenommen werden.

Die Ansteuerung bei vorhanden Mikrostrukturbereichen kann man elektrisch vornehmen, was in der Regel zu einer Leuchtdichterasterung der Bildelemente führt. Es kann aber auch eine optische Abbildung (z.B. Licht-/Laserstrahl bzw. projiziertes Bild) auf der Oberfläche des Flächenlichtmodulators vorgenommen werden, wobei dann der Flächenlichtmodulator als Lichtverstärker wirkt und hier eine rasterfreie und somit auch eine im Bildelement strukturfreie Leuchtdichte erzeugbar ist.

Es könnte auch eine zu verstärkende "Blendenkonfiguration" mit einem LCD-Display erzeugt werden, welche dann mit dem Flächenlichtmodulator verstärkt und mit einem optischen System entsprechend abgebildet wird.

Die Blendenwirkung des Flächenlichtmodulators ist oben lediglich zu Betrachtungsund zu Messzwecken beschrieben. Ein derartiger Flächenlichtmodulator kann jedoch auch zur Strahlformung eines Licht- bzw. Laserstrahls zur Behandlung am Auge verwendet werden.

Anstatt den oben beschriebenen Flächenlichtmodulator zur Beleuchtung der Augenhornhaut bzw. des Augenhintergrundes zu verwenden, kann er auch als einstellbare Blende für einen behandelnden Licht- bzw. Laserstrahl verwendet werden.

Mit den oben beschriebenen und in den **Figuren 2** bis **5** skizzierten ophthalmologischen Geräten lassen sich neben einer Reihe von Anwendungen auch Glaucoma-Patienten erfolgreich untersuchen. Es können beispielsweise mit den oben beschriebenen Flächenlichtmodulatoren Ringstrukturen erzeugt werden, welche dann mit Lichtquellen unterschiedlicher Farbe beleuchtet werden. Man kann z.B. einen blauen leuchtenden Ring erzeugen, der auf die Augennetzhaut abgebildet wird. Der Patient wird dann gefragt, was er erkennt und wo der Ring unterbrochen ist. Aus der Lage fehlender (unterbrochener) Ringsegmente kann dann auf die örtliche Lage von Retinaschädigungen geschlossen werden. Eine analoge Messung wird man anschliessend mit einem weissen oder andersfarbigen Ring vornehmen. Schädigungen von Bereichen auf der Retina sind nämlich nicht für sämtliche Farbempfindungen des Patienten gleich. Selbstverständlich kann bei dieser Untersuchung auch die Leuchtdichte auf der Retina eingestellt und verändert werden. Ein blauer Ring, d.h. eine kurzwellige Strahlung wird deshalb verwendet, da bei einer beginnenden Retinaschädigung zuerst die Blauempfindlichkeit verringert wird.

Mit den oben beschriebenen Geräten lassen sich auch Abbildungsfehler des Auges ermitteln und automatisch ausmessen. Mit dem Flächenlichtmodulator wird ein Punktemuster erzeugt und deren Lage auf der Retina durch Messen von zurückreflektierter Strahlung bestimmt. Bevorzugt wird diese Messung mit infraroter, also für die Patienten unsichtbarer Strahlung in einem abgedunkelten Raum vorgenommen. In diesem Fall ist nämlich die Pupille ganz geöffnet, wodurch sich Fehler, hervorgerufen durch die Augenlinse, gut ermitteln lassen.

Die Auswertung wird man bevorzugt mit einer digitalen Videokamera vornehmen, welche beispielsweise in den Strahlengang eines Beobachtungsmikroskops eingekoppelt ist. Die Messdaten sowie die automatisch ermittelten Resultate können gespeichert und an Bearbeitungsgeräte weiter gegeben werden. Es können somit Abbildungsfehler ermittelt werden, welche an ein Gerät zur Augenlinsenablation weitergegeben werden.

Werden unterschiedliche Wellenlängen, bevorzugt wenigstens drei unterschiedliche Wellenlängen zum Ausmessen verwendet, kann die chromatische Aberation des Auges bestimmt werden.

Es kann somit unmittelbar an das Ausmessen eine entsprechende Behandlung vorgenommen werden. Auch kann ein Ausmessen während der Behandlung laufend vorgenommen werden.

## Patentansprüche

1. Ophthalmologisches Gerät, insbesondere Spaltlampengerät, mit einer Betrachtungseinheit **(3)** und einer Beleuchtungseinheit **(5),** von der ein Lichtstrahl **(9)** zur Augenbetrachtung aussendbar ist, dessen flächige geometrische Querschnittsabmessung mit einem keine mechanischen Einstellmittel ausweisenden Flächenlichtmodulator **(29, 41, 50, 60),** dessen örtliche optische Eigenschaften änderbar und wieder rückänderbar sind, einstellbar ist, um einer auf dem Flächenlichtmodulator **(29, 41; 50; 60)** auftreffenden Strahlung **(9)** eine vorgegebene geometrische Querschnittsabmessung bzw. vorgebbare flächige Intensitätsverteilung als Bildinformation einzuprägen, **dadurch gekennzeichnet, dass** die optischen Eigenschaften des Flächenlichtmodulators **(29, 41; 50; 60)** durch Bestrahlung mit einer sich von der Beleuchtungsstrahlung **(32.3)** unterscheidenden zweiten Strahlung **(51)** änderbar sind, damit die mit dem Flächenlichtmodulator **(29, 41; 50; 60)** erzeugte Bildinformation rasterfreie Bildteilbereiche hat.

2. Gerät nach Anspruch 1, **dadurch gekennzeichnet, dass** die optischen Eigenschaften auf bzw. im Flächenlichtmodulator **(29, 41; 50; 60)** ferngesteuert durch wenigstens ein elektrisches Signal bevorzugt in einstellbaren Ortsverteilungen erzeug- und/oder änderbar sind.

3. Gerät nach Anspruch 1 oder 2, **dadurch gekennzeichnet, dass** die änderbaren optischen Eigenschaften örtlich vorgebbare Phasenlaufzeiten bzw. Phasendrehungen für die Beleuchtungsstrahlung des Flächenlichtmodulators sind.

4. Gerät nach Anspruch 1 oder 2, **dadurch gekennzeichnet, dass** die änderbaren optischen Eigenschaften über der Fläche des Flächenlichtmodulators örtlich vorgebbare Reflexionsfaktoren bzw. -richtungen für die Beleuchtungsstrahlung sind.

5. Gerät nach einem der Ansprüche 1 bis 4, **dadurch gekennzeichnet, dass** die änderbaren optischen Eigenschaften über der Fläche des Flächenlichtmodulators örtlich vorgebbare Streufaktoren für die Beleuchtungsstrahlung sind.

6. Gerät nach einem der Ansprüche 1 bis 5, **dadurch gekennzeichnet, dass** die örtlichen optischen Eigenschaften des Flächenlichtmodulators **(29, 41; 50; 60)** derart einstellbar sind, dass als flächige Intensitätsverteilung ein in der Spaltbreite und/oder in der Spalthöhe einstellbarer Spalt entsteht, dessen Spaltbild und/oder der ihn umgebende Bereich bevorzugt rasterfrei ausgebildet ist bzw. sind.

7. Gerät nach einem der Ansprüche 1 bis 6, **dadurch gekennzeichnet, dass** die örtlichen, optischen Eigenschaften des Flächenlichtmodulators **(29, 41; 50; 60)** derart einstellbar sind, dass als flächige Intensitätsverteilung eine geometrische Figur mit rasterfreien Bildelementen entsteht, wobei die Figur mit einer optischen Einheit der Beleuchtungsoptik auf das Auge **(1)** und/oder auf den Augenhintergrund abbildbar ist, um beispielsweise die Wölbung der Hornhaut bzw. des Augenhintergrundes auszumessen.

8. Gerät nach Anspruch 7, **dadurch gekennzeichnet, dass** die geometrische Figur netzartig angeordnete Bildelemente aufweist, welche beispielsweise zur Topographiebestimmung der Augenvorderfläche verwendbar sind.

9. Gerät nach Anspruch 7, **dadurch gekennzeichnet, dass** die geometrische Figur als Bildelement wenigstens einen Ring aufweist, wobei jeder Ring an einem beliebigen Ort des Flächenlichtmodulators erzeugbar ist, und jeder Ring mit wenigstens einer vorgebbaren Wellenlänge einer auftreffenden Strahlung bestrahlbar ist, um auf der Retina des Auges wenigstens einen farbigen Ring oder mehrere unterschiedlich farbige Ringe, insbesondere nach einander abzubilden, um beispielsweise örtliche Retinaschädigungen ermitteln zu können.

10. Anordnung mit einer Augenbehandlungseinrichtung, insbesondere einer Laserbehandlungseinrichtung, einem ophthalmologischen Gerät nach Anspruch 8 oder 9 und einer Datenverarbeitungseinheit, wobei die Betrachtungseinheit eine Bilderkennungseinheit hat, mit der die Figur auf der Retina erkenn- und auswertbar ist, die ausgewerteten Daten an die Datenverarbeitungseinheit übermittelbar sind und diese die Behandlungseinheit zur Augenbehandlung steuert.

## Claims

1. Ophthalmological apparatus, especially a slit lamp apparatus, having an observation unit (3) and an illuminating unit (5), from which a light beam (9) is emittable in order to observe the eye, the planar geometric cross-sectional dimension of said light beam is adjustable with a spatial light modulator (29, 41, 50, 60) having no mechanical adjustment means and the spatial optical properties of which are alterable and again reversible, in order to confer a predetermined geometric cross-sectional dimension or predeterminable two-dimensional intensity distribution, in the form of image information, on the beam emission (9) incident upon the spatial light modulator (28, 41; 50; 60), **characterised in that** the optical properties of the spatial light modulator (29, 41; 50; 60) are alterable by irradiation with a second beam emission (51) differing from the illumination beam emission (32.3), so that the image information generated with the spatial light modulator (29, 41; 50; 60) has raster-free image portions,

2. Apparatus according to claim 1, **characterised in that** the optical properties on or in the spatial light modulator (29, 41; 50; 60) are arranged to be generated and/or altered remotely, preferably In adjustable local distributions, by at least one electrical signal.

3. Apparatus according to claim 1or 2, **characterised in that** the alterable optical properties are locally predeterminable phase delays or phase angle rotations for the illumination beam emission of the spatial light modulator.

4. Apparatus according to claim 1 or 2, **characterised in that** the alterable optical properties are reflection factors and reflection directions for the illumination beam emission that are locally predeterminable over the area of the spatial light modulator.

5. Apparatus according to any one of claims 1 to 4 **characterised in that** the alterable optical propertiesare coefficients of dispersion for the illumination beam emission that are locally predeterminable over the area of the spatial light modulator.

6. Apparatus according to any one of claims 1 to 5, **characterised in that** the spatial optical properties of the spatial light modulator (29; 41; 50; 60) are adjustable such that, as two-dimensional intensity distribution, a slit adjustable in slit width and/or slit height is produced, the slit image of which and/or the region surrounding it are/is preferably of raster-free structure.

7. Apparatus according to any one of claims 1 to 6, **characterised in that** the spatial optical properties of the spatial light modulator (29; 41; 50; 60) are adjustable such that, as two-dimensional intensity distribution, a geometric figure having raster-free image elements is produced, wherein the figure is mappable using an optical unit of the illuminating optics onto the eye (1) and/or onto the fundus of the eye, in order, for example, to evaluate the convexity of the cornea or the fundus of the eye.

8. Apparatus according to claim 7, **characterised in that** the geometric figure comprises image elements arranged in the manner of a net, which are useable, for example, for determining the topography of the front surface of the eye.

9. Apparatus according to claim 7, **characterised in that** the geometric figure comprises as image element at least one ring, wherein each ring is producible at any location of the spatial light modulator, and each ring can be irradiated with at least one predeterminable wavelength of an incident beam emission in order to map on the retina of the eye at least one coloured ring or several differently coloured rings, especially in succession, in order, for example, to be able to detect local damage to the restina.

10. Arrangement having an eye treatment device, especially a laser therapy device, an ophthalmological apparatus according to claim 8 or 9 and a data processing unit, wherein the observation unit has an image-recognition unit with which the figure on the retina is recognisable and analysable, the analysed data is transferrable to the data processing unit and this controls the treatment unit for treatment of the eye.

## Revendications

1. Appareil ophtalmologique, en particulier appareil à lampe à fente, avec une unité d'examen (3) et une unité d'éclairage (5) à partir de laquelle peut être émis un faisceau d'éclairage (9) pour l'examen de l'oeil dont la dimension de section géométrique de surface peut être réglée avec un modulateur de lumière en surface (29, 41, 50, 60) ne comprenant pas de moyens mécaniques de réglage et dont les caractéristiques optiques locales sont modifiables et remodifiables en sens inverse pour imprimer, en tant qu'information d'image, à un rayonnement (9) arrivant au modulateur de lumière en surface (29, 41 ; 50 ; 60) une dimension de section géométrique imposée ou une répartition d'intensité en surface pouvant être imposée, **caractérisé en ce que** les caractéristiques optiques du modulateur de lumière en surface (29, 41 ; 50 ; 60) peuvent être modifiées par l'exposition à un deuxième rayonnement (51) se distinguant du rayonnement d'éclairage (32.3), afin que l'information d'image générée par le modulateur de lumière en surface (29, 41 ; 50 ; 60) possède des zones partielles d'images exemptes de trames.

2. Appareil selon la revendication 1, **caractérisé en ce que** les caractéristiques optiques sur ou dans le modulateur de lumière en surface (29, 41 ; 50 ; 60) peuvent être générées et/ou modifiées par télécommande par au moins un signal électrique, de préférence dans des circuits de répartitions locaux réglables.

3. Appareil selon la revendication 1 ou 2, **caractérisé en ce que** les caractéristiques optiques. modifiables sont des temps de propagation de phases ou des rotations de phases pouvant être prédéterminés localement pour le faisceau d'éclairage du modulateur de lumière en surface.

4. Appareil selon la revendication 1 ou 2, **caractérisé en ce que** les caractéristiques optiques modifiables sont des facteurs ou des directions de réflexion pour le faisceau d'éclairage pouvant être imposés localement par l'intermédiaire de la surface du modulateur de lumière en surface.

5. Appareil selon l'une des revendications 1 à 4, **caractérisé en ce que** les caractéristiques optiques modifiables sont des facteurs de diffusion pour le faisceau d'éclairage pouvant être imposés localement par l'intermédiaire de la surface du modulateur de lumière en surface.

6. Appareil selon l'une des revendications 1 à 5, **caractérisé en ce que** les caractéristiques optiques locales du modulateur de lumière en surface (29, 41 ; 50 ; 60) peuvent être réglées de telle manière que, comme répartition d'intensité en surface, se constitue une fente dont la largeur et/ou la hauteur peuvent être réglées et dont l'image de fente et/ou la zone qui l'entoure est (sont) de préférence réalisée(s) sans trames.

7. Appareil selon l'une des revendications 1 à 6, **caractérisé en ce que** les caractéristiques optiques locales du modulateur de lumière en surface (29, 41 ; 50 ; 60) peuvent être réglées de telle manière qu'une figure géométrique avec des éléments d'images sans trames soient obtenues comme répartition d'intensité en surface, la figure étant, avec une unité optique de l'appareil optique d'éclairage, reproductible sur l'oeil (1) et/ou sur le fond de l'oeil pour par exemple mesurer la courbure de la cornée ou du fond de l'oeil.

8. Appareil selon la revendication 7, **caractérisé en ce que** la figure géométrique comprend des éléments d'images disposés de manière réticulée, qui sont par exemple utilisables pour la détermination de la topographie de la surface avant de l'oeil.

9. Appareil selon la revendication 7, **caractérisé en ce qu'**en tant qu'élément d'image la figure géométrique comprend au moins un cercle, chaque cercle pouvant être généré à un endroit quelconque du modulateur de lumière en surface et chaque cercle pouvant être soumis à un rayonnement avec au moins une longueur d'onde du rayonnement incident pouvant être imposée, et ceci pour pouvoir former sur la rétine de l'oeil, en particulier successivement, au moins un cercle de couleur ou plusieurs cercles de couleurs différentes, afin de pouvoir détecter par exemple des détériorations locales de la rétine.

10. Ensemble avec un dispositif de traitement de l'oeil, en particulier un dispositif de traitement au laser, un appareil ophtalmologique selon la revendication 8 ou 9 et une unité de traitement de données, l'unité d'examen disposant d'une unité de reconnaissance d'image à l'aide de laquelle la figure sur la rétine est reconnaissable et exploitable, les données exploitées étant transmissibles à l'unité de traitement de données et celle-ci pouvant commander l'unité de traitement en vue du traitement de l'oeil.
